# EUROPÄISCHE PATENTSCHRIFT

(11) **EP 4 407 305 B1**
(45) Veröffentlichungstag und Bekanntmachung des Hinweises auf die Patenterteilung: **01.04.2026**
(21) Anmeldenummer: 24152802.5
(22) Anmeldetag: 19.01.2024
(51) Int. Cl.: G01N 27/08, A61M 1/00, G01R 27/00

(54) **LEITFÄHIGKEITSSONDE**
CONDUCTIVITY PROBE
SONDE DE CONDUCTIVITÉ

(30) Priorität: 26.01.2023 DE 102023101900
(43) Veröffentlichungstag der Anmeldung: 31.07.2024
(73) Patentinhaber: B. Braun Avitum AG, 34212 Melsungen (DE)
(72) Erfinder: BERTRAM, Rolf, 34286 Bergheim (DE)
(74) Vertreter: Winter, Brandl - Partnerschaft mbB

(56) Entgegenhaltungen:
- KR-A- 20190 050 654
- US-A1- 2018 120 247
- US-A1- 2019 125 440
- ANONYMOUS: "Gardena Reparator 19mm 3/4'' Schlauchverbinder - Eisenwaren-Hingst", 1 January 2023 (2023-01-01), XP093168923, Retrieved from the Internet <URL:https://eisenwaren-hingst.com/produkt/gardena-reparator-19mm-3-4-schlauchverbinder/>
- ANONYMOUS: "Flex-Verbindungsschlauch 14,9 mm (G 3/8) x 16,7 mm (R 3/8) x 100 mm kaufen bei OBI", 1 January 2023 (2023-01-01), XP093169135, Retrieved from the Internet <URL:https://www.obi.at/flexschlaeuche/flex-verbindungsschlauch-14-9-mm-g-3-8-x-16-7-mm-r-3-8-x-100-mm/p/5652995>

## Beschreibung

### Technisches Gebiet

Die vorliegende Offenbarung eine Leitfähigkeitssonde, in der Abstandhalter zusammen mit Elektroden in abwechselnder Reihenfolge zusammengesetzt werden. Die Elektroden werden zur Leitfähigkeitsmessung insbesondere bei der Herstellung von Dialysierflüssigkeit eingesetzt. Dazu bilden die Durchgangsausnehmungen der Elektroden zusammen mit den dazwischen angeordneten Durchgangskanälen der Abstandhalter einen Messkanal der Leitfähigkeitssonde aus. Dieser Messkanal wird insbesondere bei der Herstellung der Dialysierflüssigkeit von deren Komponenten Permeat und Bikarbonat-Konzentrat oder später von der fertigen Dialysierflüssigkeit durchströmt.

### Stand der Technik

Aus dem hausinternen Stand der Technik sind derartige Abstandhalter bekannt, die den zentralen sich in Strömungsrichtung der Dialysierflüssigkeit oder der genannten Komponenten erstreckenden Durchgangskanal und zwei quer dazu angeordnete Anlageflächen aufweisen. Je nach Verbauort des Abstandhalters in der Leitfähigkeitssonde liegen an den beiden Anlageflächen eines Abstandhalters entweder jeweils eine Stirnseite einer benachbarten Elektrode an, oder es liegt nur an einer der Anlageflächen eine Elektrode an, während an der anderen Anlagefläche ein Abschlussflansch der Leitfähigkeitssonde anliegt.

Derartige Abstandhalter werden gemäß dem hausinternen Stand der Technik zur dichtenden Anlage mit den Elektroden bzw. mit den Abschlussflanschen mittels mehrerer durchgehender Zuganker verspannt. Diese Zuganker erstrecken sich parallel zum Messkanal am Außenumfang der Abstandhalter und der Elektroden und durchsetzen die beiden Abschlussflansche. Wenn die Zuganker gespannt werden, spannen sie den geschichteten Aufbau von Elektroden und Abstandhaltern dichtend gegeneinander.

Nachteilig an Leitfähigkeitssonden, die mit derartigen Abstandhaltern zusammengesetzt sind, ist der hohe Aufwand für die nachträgliche Behebung eines Montagefehlers, z.B. für die Ergänzung oder Korrektur einer Dichtung, denn es müssen in diesem Fall die durchgehenden Zuganker entspannt und entfernt werden, wodurch der gesamte geschichtete Aufbau wieder auseinanderfällt. Nach der Behebung des Montagefehlers müssen alle Einzelteile (Elektroden, Abstandhalter und Dichtringe) erneut korrekt zusammengesetzt werden. Dabei ist der Aufwand für die Behebung eines Montagefehlers natürlich abhängig von der Anzahl der Elektroden und Abstandhalter.

US 2019/0125440 A1 offenbart einen Elektrophysiologie-Katheter mit modularer Elektrodenstruktur, bestehend aus mehreren nichtleitenden Zylinderelementen und dazwischen angeordneten Elektroden. Die nichtleitenden Zylinder sind über einen Schnappmechanismus miteinander verbunden.

KR 20190050654 offenbart eine modulare Leitfähigkeitssonde, die aus isolierenden und leitenden Segmenten besteht, welche ineinander gesteckt sind.

### Kurzbeschreibung der Offenbarung

Aufgabe der vorliegenden Offenbarung ist es daher, eine Leitfähigkeitssonde mit mindestens einem Abstandhalter und mit mindestens zwei Elektroden zu schaffen, bei der der oben genannte Nachteil behoben ist.

Die Aufgabe wird gelöst durch eine Leitfähigkeitssonde mit der Merkmalskombination gemäß Anspruch 1.

Wie vorstehend bereits ausgeführt wurde, müssen für eine Leitfähigkeitsmessung wenigstens zwei Elektroden vorzugsweise gemäß der vorliegenden Offenbarung in einem (vor-)bestimmten Axialabstand zueinander (in einem Leitungssystem) angeordnet werden, wofür bevorzugt ein Abstandshalter vorgesehen ist.

Der Abstandhalter gemäß der Offenbarung wird hierfür zusammen mit Elektroden in eine Leitfähigkeitssonde beispielsweise eines Dialysegerätes insbesondere eines Hämodialysegeräts eingebaut und ist als solches dafür ausgelegt, alleinig oder in Zusammenschluss mit weiteren/zusätzlichen Abstandshaltern gleichen Aufbaus die Leitfähigkeits-Messelektroden in ein Leitungssystem vorzugsweise Dialysierflüssigkeits-Leitungssystems des Dialysegeräts zu integrieren. Der Abstandhalter weist einen Durchgangskanal mit zwei axialbeabstandeten Mündungen auf, wobei an jeder Mündung ein Anlage-/Montagebereich für eine Leitfähigkeitsmesselektrode vorzugsweise gemäß vorstehender Offenbarung oder einen weiteren Abstandshalter vorgesehen ist, wobei der jeweilige Anlage-/Montagebereich eine die jeweilige Mündung umgebende (Elektroden-)Anlagefläche aufweist. An den beiden Anlagebereichen ist jeweils eine eigene (unabhängig und separat betätigbare und funktionierende) Spannvorrichtung vorgesehen, derart, dass die beiden Spannvorrichtungen des Abstandhalters unabhängig voneinander aktivierbar und deaktivierbar sind. Bei ihrer Aktivierung sind die beiden Spannvorrichtungen des Abstandhalters unabhängig voneinander in Wirkverbindung beispielsweise mit einer jeweiligen entsprechenden Spannvorrichtung eines Anlagebereiches eines weiteren Abstandhalters oder Abschlussflansches oder einer Elektrode vorzugsweise gemäß der vorliegenden Offenbarung bringbar.

Aus Gründen der korrekten Formulierung der Merkmale nur genau eines beanspruchten Abstandhalters ist der Begriff "Spannvorrichtung" als ein Teil von zwei Teilen zu verstehen, die zusammen für eine Einspannung einer Elektrode nötig sind.

Bei einer fertigungstechnisch einfachen Ausgestaltung des Abstandhalters ist der Durchgangskanal gerade und dabei normal bzw. senkrecht zu den beiden Anlageflächen ausgerichtet.

Nach dem gleichen Prinzip ist auch eine Anordnung offenbart, die zwei Abstandhalter und dazwischen eine Dialyse-Leitfähigkeitsmesselektrode aufweist, wobei jeder Abstandhalter einen axialen Durchgangskanal und zwei quer dazu angeordnete Anlageflächen aufweist, wobei an zwei aufeinander zuweisenden Anlageflächen der beiden Abstandhalter jeweils eine Stirnseite der Dialyse-Leitfähigkeitsmesselektrode anliegt. Die Dialyse-Leitfähigkeitsmesselektrode hat eine Einspannung, die aus zwei Spannvorrichtungen gebildet ist, die an den beiden aufeinander zuweisenden Anlagebereichen der beiden Abstandhalter gebildet sind.

Mit dem definierten Abstandshalter und mit der definierten Anordnung ist die Dialyse-Leitfähigkeitsmesselektrode unabhängig von weiteren Einspannungen der betroffenen Leitfähigkeitssonde einspannbar bzw. eingespannt und kann, wenn z.B. ein Leck festgestellt wird, nur an der betroffenen Einspannung wieder gelöst oder geöffnet werden, z.B. um einen fehlenden/beschädigten Dichtring zwischen der Anlagefläche des Abstandhalters und der Stirnfläche der Elektrode zu ergänzen oder um seinen Sitz zu korrigieren.

Weiterhin hat die individuelle Einspannung gemäß der Offenbarung gegenüber der durchgehenden verketteten Einspannung des Standes der Technik den Vorteil, dass auf die individuelle Einspannung nur die Kräfte von zwei Dichtringen wirkt, die zusammen mit der Elektrode zwischen den aufeinander zuweisenden Anlageflächen zweier Abstandhalter eingespannt sind.

Die beiden Anlageflächen haben vorzugsweise jeweilige zumindest abschnittsweise umlaufende Außenränder, um die anliegende Elektrode zu positionieren und in Position zu halten. Die beiden Außenränder können rund oder auch eckig (z.B. quadratisch) sein. Entsprechend hat die Elektrode einen Hauptkörper mit einem runden oder eckigen (z.B. quadratischen) Querschnitt.

Vorzugsweise ist an einem der beiden Außenränder oder an beiden Außenrändern eine Ausnehmung (Kerbe) für einen elektrischen Kontakt der Elektrode gebildet. Wenn die beiden Außenränder nicht komplett umlaufend sind, können sie z.B. durch die Ausnehmung (Kerbe) unterbrochen sein.

Bei einer besonders bevorzugten Ausgestaltung ist an beiden Anlageflächen und an beiden Mündungen jeweils ein Dichtring angeordnet. Damit benötigen die Elektroden (ihrerseits) keine Dichtringe, so dass die Elektroden mit verringertem Außendurchmesser hergestellt werden können. Weiterhin können die Stirnseiten der Elektroden ohne Nut für Dichtringe und/oder flächig ausgeführt werden.

Bei einem ersten Dichtkonzept des Abstandhalters können die beiden Dichtringe stoffschlüssig, also chemisch an der Anlagefläche befestigt sein.

Bei einem zweiten Dichtkonzept können die beiden Dichtringe formschlüssig an der Anlagefläche befestigt sein.

Beide genannten Dichtkonzepte haben den Vorteil, dass durch die (insbesondere beidseitige) Befestigung der Dichtringe am Abstandhalter beim Zusammenbau der übergeordneten Leitfähigkeitssonde die Teilanzahl deutlich verringert ist, und dass Fehler wegen fehlender oder nicht korrekt eingesetzter Dichtringe ausgeschlossen sind.

Bei einer besonders bevorzugten Ausgestaltung des zweiten Dichtkonzepts ist an beiden Anlageflächen jeweils ein Dichtring angeordnet, der die jeweilige Mündung des Durchgangskanals vollumfänglich umgibt. Zur Herstellung des Formschlusses sind die beiden Dichtringe gemeinsam mit mindestens einem Verbindungselement/Steg zu einem (stoff-)einstückigen Dichtbauteil insbesondere aus Silikon oder einem anderen Dichtwerkstoff gebildet. Das mindestens eine Verbindungselement erstreckt sich axial zwischen den beiden Dichtringen.

Bei einer Weiterbildung des Abstandhalters sind die beiden Anlagebereiche jeweils von einem Flansch gebildet, wobei sich zwischen den beiden Flanschen ein Hals mit gegenüber den beiden Flanschen vermindertem Durchmesser erstreckt. In dem Hals ist ein mittlerer Abschnitt des Durchgangskanals gebildet.

Bei einem Ausführungsbeispiel umfasst oder ist die Einspannung der Elektrode eine Ringschnappverbindung. Damit umfasst oder ist die beanspruchte Spannvorrichtung des Abstandhalters eine Seite dieser Ringschnappverbindung.

Die Ringschnappverbindung ist fertigungstechnisch einfach und bietet dabei maximalen Halt zwischen den beiden betroffenen Abstandhaltern, wenn sie kreis- oder kreisbogenförmig ausgestaltet ist und sich vollumfänglich oder weitgehend oder abschnittsweise an den beiden aufeinander zuweisenden Außenrändern erstreckt.

Vorzugsweise weist die Ringschnappverbindung einen umlaufenden äußeren Wulst an dem einen Außenrand und eine umlaufende innere Nut an dem anderen Außenrand auf.

Die Geometrie der Ringschnappverbindung könnte alternativ auch eine lösbare oder nicht lösbare Rasthakengeometrie sein.

Bei einem weiteren Ausführungsbeispiel umfasst oder ist die Einspannung der Elektrode eine Clipsverbindung. Damit umfasst oder ist die beanspruchte Spannvorrichtung des Abstandhalters eine Seite dieser Clipsverbindung. Vorzugsweise ist die Clipsverbindung mehrteilig und am Umfang verteilt.

Die Einspannung kann auch eine Bügelverbindung sein, die mindestens einen Bügel umfasst. Der Bügel hat zwei Schenkel, von den einer in ein Bügelloch des Anlagebereiches des einen Abstandhalters eingesetzt ist, während der andere Schenkel in ein entsprechendes Bügelloch des benachbarten Anlagebereiches des anderen Abstandhalters eingesetzt ist.

Gemäß einem bevorzugten Ausführungsbeispiel wird mit den beiden aufeinander zuweisenden Spannvorrichtungen der beiden Abstandhalter eine Bügelverbindung geschaffen, die zwei Bügel und vier Bügellöcher hat, wobei die beiden Bügel auf einander gegenüberliegenden Seiten des Durchgangskanals angeordnet sind.

Die drei genannten Einspannungen können auch beliebig kombiniert werden. So kann die Bügelverbindung mit der Ringschnappverbindung oder mit der Clipsverbindung kombiniert werden.

Die Leitfähigkeitssonde gemäß der Offenbarung hat mindestens einen, (vorzugsweise mehrere) vorbeschriebenen Abstandhalter und mindestens zwei (vorzugsweise mehr als zwei) Elektroden, wobei die Anzahl der Elektroden um eins größer ist, als die Anzahl der Abstandhalter. An den beiden äußersten Elektroden eines Messkanals der Leitfähigkeitssonde ist jeweils innenseitig der genau eine Abstandhalter oder ein äußerster Abstandhalter angeordnet während außenseitig ein Abschlussflansch der Leitfähigkeitssonde angeordnet ist. Auch die beiden Abschlussflansche weisen jeweils einen Anlagebereich mit einer Anlagefläche auf, so dass die beiden äußersten Elektroden jeweils zwischen den beiden aufeinander zuweisenden Anlageflächen des genau einen oder des äußersten Abstandhalters und des Abschlussflansches dichtend eingespannt sind. Auch an den Anlagebereichen der beiden Abschlussflansche ist jeweils eine Spannvorrichtung vorgesehen. Auch diese Spannvorrichtungen sind unabhängig voneinander aktivierbar und deaktivierbar, und bei ihrer Aktivierung unabhängig voneinander in Wirkverbindung mit der benachbarten Spannvorrichtung des Anlagebereiches des genau einen oder des äußersten Abstandhalters bringbar. Vorzugsweise entsprechen die beiden Spannvorrichtungen der beiden Abschlussflansche denjenigen des vorbeschriebenen Abstandhalters.

Damit ist jede einzelne Elektrode unabhängig von weiteren Elektroden in die Leitfähigkeitssonde eingespannt. Die Leitfähigkeitssonde kann, wenn z.B. ein Leck festgestellt wird, nur an der betroffenen Einspannung wieder gelöst oder geöffnet werden, z.B. um einen fehlenden Dichtring zwischen der Anlagefläche des Abstandhalters und der Stirnfläche der Elektrode zu ergänzen oder dessen Sitz zu korrigieren.

Am Außenumfang des mindestens einen Abstandhalters und der mindestens zwei Elektroden kann eine Überwurfhülse angeordnet sein. Diese kann aus Aluminium oder Edelstahlblech oder Stahlblech oder Kunststoff oder glasfaserverstärktem Kunststoff gefertigt sein. Die Überwurfhülse kann als Montagehilfe und/oder als Stützhülse dienen.

Die Überwurfhülse kann (z.B. über ihre volle Länge) einseitig offen sein, um dort Raum für die elektrischen Kontakte der Elektroden zu lassen.

An zumindest einem der beiden Abschlussflansche kann eine Schlauchtülle und/oder eine Sensoraufnahme und/oder ein Befestigungsmittel zur Befestigung der Leitfähigkeitssonde an dem Rahmen z.B. des Dialysegerätes gebildet sein. **In** die Sensoraufnahme kann ein Sensor für Temperatur, Druck oder Flussgeschwindigkeit eingesetzt werden.

Alternativ oder in Ergänzung kann das Befestigungsmittel auch an der Überwurfhülse gebildet oder angeordnet sein.

Die Elektrode bzw. Dialyse-Leitfähigkeitsmesselektrode gemäß der Offenbarung hat einen (Puck-artigen) Hauptkörper, der eine (axial sich erstreckende) Durchgangsausnehmung oder Kanal hat oder umgibt, die zwei (zueinander parallele bzw. parallel-beabstandete) Stirnseiten des Hauptkörpers miteinander verbindet. **In** der Durchgangsausnehmung ist ein elektrisch leitfähiger Innenmantel angeordnet, der von einer zu messender Dialysierflüssigkeit oder von deren Komponenten durchströmbar ist. **In** anderen Worten ausgedrückt ist ein den Innenmantel definierendes Innenrohr aus einem elektrisch leitfähigen Material vorgesehen, das von einem Block aus einem vorzugsweise elektrisch nichtleitenden Material umgeben ist. Der Materialblock bildet dabei einen Hauptkörper der Elektrode. Das Innenrohr ist entweder als ein zum Materialblock separat gebildetes Bauteil oder durch Beschichten der im Materialblock vorgesehenen Durchgangsausnehmung ausgebildet.

An der Außenseite, insbesondere am Außenumfang des Hauptkörpers ist ein elektrischer Kontakt angeordnet, der sich vorzugsweise radial von der Durchgangsausnehmung oder dem Innenmantel weg erstreckt und mit dem Innenmantel elektrisch verbunden ist. Der Innenmantel und der elektrische Kontakt sind vorzugsweise gemeinsam aus einem ersten elektrisch leitfähigen Material gebildet. Der Hauptkörper ist vorzugsweise ein von dem Innenmantel und dem elektrischen Kontakt getrenntes Trägerbauteil, das aus einem zweiten, vorzugsweise elektrisch nichtleitfähigen Material (weiter vorzugsweise aus Vollmaterial) gefertigt ist.

**In** vorteilhafter Weise besteht die Elektrode also aus einer elektrisch leitfähigen Hülse (Innenrohr/Innenmantel) mit einer daran angeformten oder fixierten, elektrisch leitfähigen Fahne oder Litze, die gemeinsam von einem elektrisch isolierenden Materialblock/Trägerbauteil ummantelt/umgeben sind, derart, dass die Hülse innenwandig zumindest teilweise frei exponiert (nicht vom Materialblock abgedeckt) verbleibt sowie deren axiale Stirnseiten zumindest teilweise nach außen offen sind (und damit die Hülse durchströmbar ist) und die Fahne oder Litze zumindest an deren freiem Endabschnitt eine (von außen frei zugängliche) elektrische Anschlussstelle bildet.

Die Herstellungskosten der offenbarungsgemäßen Elektrode sind gegenüber dem Stand der Technik vermindert, da das zweite (elektrisch isolierende) Material des Trägerbauteils beispielsweise ein Kunststoff, eine Keramik oder dergleichen Isolationsmaterial sein kann und daher weniger teuer als das Graphit des Standes der Technik ist und einfach, beispielsweise durch Spritzgussformen hergestellt werden kann. Weiterhin ist die Fertigung der elektrisch leitfähigen Bestandteile der Elektrode vereinfacht da diese im Wesentlichen nur aus Innenmantel und Fahne / Litze bestehen. Es bieten sich mit dem Hauptkörper gemäß der Offenbarung auch vereinfachte Möglichkeiten zusätzliche Dichtringe an den beiden axialbeabstandeten Stirnseiten des Hauptkörpers/Materialblocks/Trägerbauteils zu befestigen, wodurch die Anzahl der Einzelteile der übergeordneten Leitfähigkeitssonde vermindert ist. So ist es beispielsweise möglich, zwei Dichtringe über axiale Stege vorzugsweise stoffeinstückig in einem vorbestimmten Axialabstand zueinander zu verbinden und anschließend vom Materialblock beispielsweise durch Spritzgießen zu umschließen. Auf diese Weise werden die Dichtringe fest und sicher am Materialblock (durch mechanische Kopplung und nicht nur per Klebwirkung) gehalten.

Damit ist der Zusammenbau der Elektrode gemäß der Offenbarung vereinfacht.

Der Hauptkörper und der in der Durchgangsausnehmung angeordnete oder gebildete Innenmantel können in per se aus dem Stand der Technik bekannter Weise kreiszylindrisch und konzentrisch zueinander sein. Es ist aber auch möglich, dass der Hauptkörper (und ggf. der Innenmantel) eine hierzu abweichende Form hat, z.B. quaderförmig ist. Dabei kann der Querschnitt des Hauptkörpers (quer zur Durchflussrichtung oder Mittelachse des Innenmantels) quadratisch sein. Dies kann einer vereinfachten Montage und/oder Lagepositionierung dienen, wenn zwei derartige Elektroden über zumindest einen Abstandhalter zueinander beabstandet montiert werden. Ggf. kann die Querschnittsform des Innenmantels von der Querschnittsform des Hauptkörpers abweichen.

Gemäß dem Stand der Technik ist der elektrische Kontakt eine Litze, die als Stampfkontakt ausgebildet bzw. befestigt ist. Dem gegenüber ist es offenbarungsgemäß bevorzugt, wenn der elektrische Kontakt (Fahne) ein Flachkontakt und/oder ein Steckkontakt ist. Damit ist die Herstellung des elektrischen Kontakts vereinfacht. Der elektrische Kontakt kann auch ein Durchgangsloch/-Schlitz zur leichteren Befestigung einer separaten Anschlusslitze haben, die nicht Bestandteil der Elektrode ist.

Gemäß einem ersten Dichtkonzept ist an zumindest einer der Stirnseiten der eine Dichtring angeordnet, der eine an der Stirnseite gebildete Mündung der Durchgangsausnehmung vollumfänglich umgibt, und der stoffschlüssig, also chemisch an der Stirnseite befestigt ist. Dabei wird es besonders bevorzugt, wenn an beiden Stirnseiten und an beiden Mündungen jeweils ein derartiger Dichtring angeordnet ist. Dann sind alle Dichtungen bereits mit der Fertigung des Hauptkörpers vormontiert und die Anzahl der Einzelteile der übergeordneten Leitfähigkeits-Messsonde/Messzelle ist reduziert, wodurch deren Montage vereinfacht ist. Insbesondere ist die Gefahr ausgeschlossen, dass beim Zusammenbau der Leitfähigkeitssonde ein Dichtring vergessen oder nicht korrekt positioniert wird.

Gemäß einem zweiten Dichtkonzept kann der mindestens eine Dichtring auch formschlüssig an der Stirnseite befestigt sein.

Bei einer bevorzugten konkreten Ausgestaltung des zweiten Dichtkonzeptes ist an beiden Stirnseiten jeweils ein Dichtring angeordnet, der die jeweilige an der Stirnseite gebildete Mündung der Durchgangsausnehmung vollumfänglich umgibt. Die beiden Dichtringe bilden gemeinsam mit mindestens einem Verbindungselement/Steg ein (stoff-)einstückiges Dichtbauteil insbesondere aus Silikon.

Im Unterschied zu der vorstehenden Beschreibung ist es alternativ möglich, dass sich das mindestens eine Verbindungselement/Steg zwischen den beiden Dichtringen durch ein als Durchgangsloch im Hauptkörper gebildetes Verbindungsloch erstreckt. Das Dichtbauteil kann in diesem Fall an den bereits (vor-)gefertigten Hauptkörper in einem Schuss gespritzt werden.

So können (gleichmäßig) am Außenumfang des Innenmantels der Durchgangsausnehmung verteilt zwei, drei oder vier Verbindungslöcher vorgesehen sein, durch die sich jeweils ein Verbindungselement des Dichtbauteils erstreckt.

Der Hauptkörper ist aus dem zweiten vorzugsweise nicht elektrisch leitfähigen Material gefertigt. Dieses kann biokompatibles PPE und PS oder PEI oder PSU sein oder zumindest enthalten.

Der Innenmantel und der elektrische Kontakt (Fahne) sind gemeinsam aus dem ersten elektrisch leitfähigen Material gebildet. Dieses kann Kupfer oder Messing, Silber oder Hartgold oder Graphit sein oder zumindest enthalten. Das erste Material insbesondere in Form von Hartgold oder Graphit kann als Beschichtung auf dem Hauptkörper aufgetragen sein.

Der elektrische Kontakt kann über einen Verbindungs-Zwischenabschnitt mit dem Innenmantel verbunden und elektrisch kontaktiert sein. Der Verbindungs-Zwischenabschnitt (nachfolgend einfach als Verbindungsabschnitt bezeichnet) dient quasi als elektrische Brücke zwischen dem radial vom Innenmantel beabstandeten und zumindest teilweise außerhalb des Hauptkörpers angeordneten elektrischen Kontakt und dem Innenmantel.

Bei einem ersten Ausführungsbeispiel der Dialyse-Leitfähigkeitsmesselektrode bilden der Innenmantel und der elektrische Kontakt ein (stoff-)einstückiges Stanz-Biege-/ Stanz-Tiefzieh-Bauteil. Der Hauptkörper ist ein Kunststoff-Spritzgussteil, das um den Innenmantel und zumindest um den Verbindungsabschnitt oder teilweise um den elektrischen Kontakt (Fahne) herumgespritzt ist.

Offenbart ist auch ein erstes Ausführungsbeispiel eines Herstellungsverfahrens einer Dialyse-Leitfähigkeitsmesselektrode, bei dem zunächst das Stanz-Biege-Bauteil aus dem ersten Material gefertigt wird, und danach der Hauptkörper aus dem zweiten Material um des Stanz-Biege-Bauteil herumgespritzt wird.

Bei einer konkreten Ausgestaltung des ersten Ausführungsbeispiels weist das Stanz-Biege-Bauteil einen den Innenmantel ausbildenden Zylinder/Hülse auf, an dem ein Flansch oder Kragen gebildet ist. Der Flansch/Kragen ist an einer der Stirnseiten des Hauptkörpers angeordnet, und der Verbindungsabschnitt/Fahne erstreckt sich radial weg von dem Flansch.

Bei dünneren Flachkontakten oder Steckkontakten (zungenförmige Kontaktfahnen) kann es von großem Vorteil sein, wenn der Hauptkörper eine (stoff-)einstückig angeformte/ausgebildete Kontaktstütze ausbildet/hat, quasi In Form einer Schiene aus elektrisch nichtleitendem Material, an welcher der elektrische Kontakt in Kontaktlängsrichtung einseitig aufgelegt ist und sich so daran abstützt (und daher nichtmehr umgebogen werden kann). Wenn der elektrische Kontakt zusätzlich das Durchgangsloch für die Litze hat, dann hat selbstverständlich auch die Kontaktstütze ein entsprechendes (deckungsgleiches) Durchgangsloch für die Litze.

Bei einem zweiten Ausführungsbeispiel der Dialyse-Leitfähigkeitsmesselektrode sind der Innenmantel und der elektrische Kontakt per MID-Technik (molded interconnect device) oder mittels einer ähnlichen Beschichtungstechnik als Beschichtung auf den Hauptkörper und auf dessen Kontaktstütze aufgebracht. Vorzugsweise bestehen der aufgetragene Innenmantel und der aufgetragene elektrische Kontakt aus Graphit oder Kupfer.

Offenbart ist auch ein zweites Ausführungsbeispiel eines Herstellungsverfahrens einer Dialyse-Leitfähigkeitsmesselektrode, bei dem zunächst der Hauptkörper aus dem zweiten Material gefertigt wird, und danach der Innenmantel und der elektrische Kontakt aus dem ersten Material auf den Hauptkörper aufgetragen werden.

Bei einer Ausgestaltung des zweiten Ausführungsbeispiels ist der Verbindungsabschnitt an einer der beiden Stirnseiten des Hauptkörpers angeordnet.

Bei einem dritten Ausführungsbeispiel der Dialyse-Leitfähigkeitsmesselektrode sind der Innenmantel und der elektrische Kontakt und der Hauptköper mittels eines additiven Verfahrens (3D-Druck) gebildet.

Offenbart ist auch ein additives Herstellungsverfahren einer Dialyse-Leitfähigkeitsmesselektrode, wobei der Innenmantel und der elektrische Kontakt aus dem ersten Material und der Hauptköper aus dem zweiten Material mittels eines Verfahrens (3D-Druck) gleichzeitig bzw. in einem Vorgang gebildet werden.

Bei einer konkreten Ausgestaltung des dritten Ausführungsbeispiels ist der Verbindungsabschnitt geschützt im Innern des Hauptkörpers angeordnet.

Das elektrisch leitende erste Material, aus dem der elektrische Kontakt und der Innenmantel und ggf. auch der Zylinder und ggf. auch der Verbindungsabschnitt gefertigt sind, kann ein nicht metallischer elektrisch leitender (moderner) Kunststoff sein.

### Kurzbeschreibung der Figuren

Fig. 1 zeigt eine Leitfähigkeitssonde gemäß der vorliegenden Offenbarung in einer Ansicht;
Fig. 2 zeigt eine Elektrode gemäß einem ersten Beispiel;
Fig. 3 zeigt eine Explosionszeichnung der Elektrode aus Fig. 2;
Fig. 4 zeigt eine Elektrode gemäß einem zweiten Beispiel in einem unfertigen Zustand;
Fig. 5 zeigt die Elektrode aus Fig. 4 im fertigen Zustand;
Fig. 6 zeigt eine Darstellung einer Elektrode gemäß einem dritten Beispiel in einem unfertigen Zustand;
Fig. 7 zeigt die Elektrode aus Fig. 6 im fertigen Zustand;
Fig. 8 zeigt die Elektrode aus Fig. 7 in einer geschnittenen Darstellung;
Fig. 9 zeigt eine lange und eine kurze Version eines ersten Ausführungsbeispiels eines Abstandhalters mit der Elektrode aus Figs. 2 und 3 in einer Explosionszeichnung;
Fig. 10 zeigt die Anordnung aus Fig. 9 im zusammengesetzten Zustand mit zwei weiteren Elektroden;
Fig. 11 zeigt eine lange und eine kurze Version eines zweiten Ausführungsbeispiels eines Abstandhalters;
Fig. 12 zeigt einen oberen und einen unteren Abschnitt der Leitfähigkeitssonde aus Fig. 1 mit den Abstandhaltern aus Fig. 11 und mit mehreren Elektroden in einer geschnittenen Darstellung.

### Beschreibung der Ausführungsbeispiele

Nachstehend werden mehrere Ausführungsbeispiele der vorliegenden Offenbarung auf der Basis der zugehörigen Figuren beschrieben.

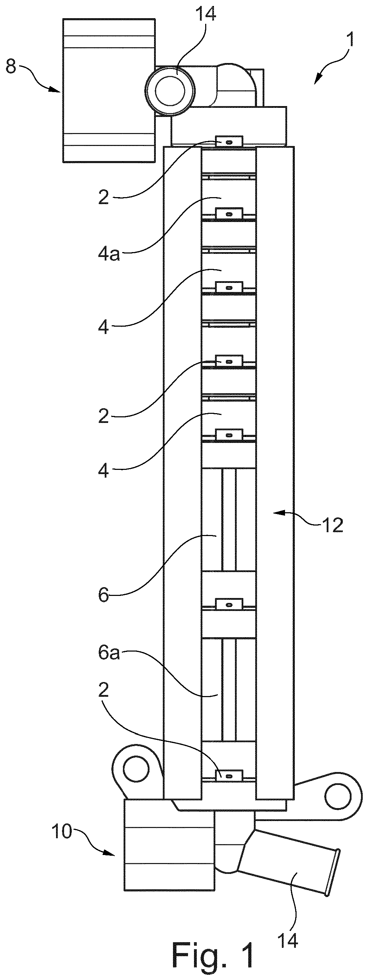

Fig. 1 zeigt eine Leitfähigkeitssonde 1. Sie hat mehrere Elektroden, von denen in Fig. 1 jeweils lediglich ein elektrischer Kontakt 2 zu erkennen ist. Die Leitfähigkeitssonde 1 wird direkt oder mittels Montageadapter (Hilfselement; Blech; spritzgegossener Träger) an einem Rahmen eines Dialysegerätes befestigt und (von einem Permeat und Bikarbonat-Konzentrat oder) von einer fertigen Dialysierflüssigkeit durchströmt.

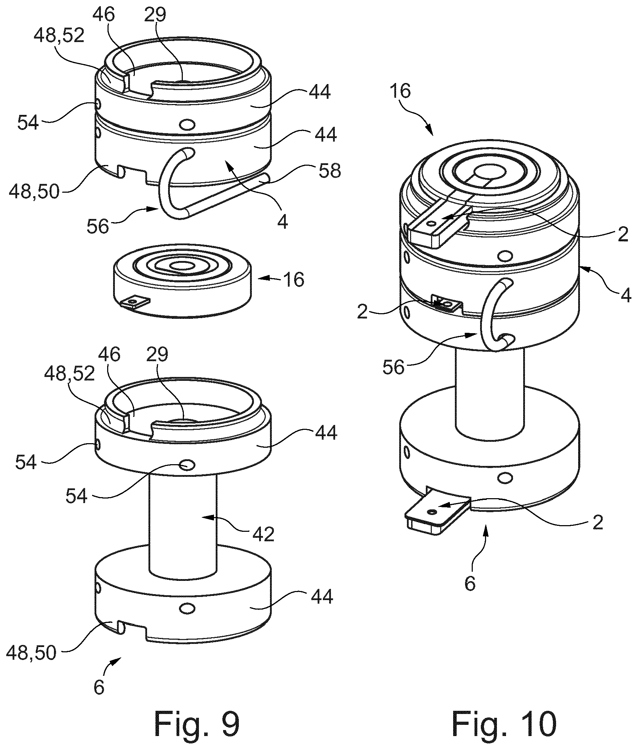

Die Elektroden werden entlang eines inneren Messkanals mit Abstandhaltern 4, 4a, 6, 6a voneinander in einem vorbestimmten Maß beabstandet. Dazu sind kürzere Abstandhalter 4, 4a und längere Abstandhalter 6, 6a vorgesehen. Mehrere Elektroden und mehrere Abstandhalter 4, 4a, 6, 6a sind abwechselnd angeordnet und mit zwei Abschlussflanschen 8, 10 verspannt. Genauer gesagt bildet der (in Fig. 1 obere) Abschlussflansch 8 direkt mit dem (in Fig. 1 darunter angeordneten) benachbarten äußersten Abstandhalter 4a eine Einspannung für die dortige äußerste Elektrode. Auf gleiche Weise bildet der (in Fig. 1 untere) Abschlussflansch 10 direkt mit dem (in Fig. 1 darüber angeordneten) benachbarten äußersten Abstandhalter 6a eine Einspannung für die dortige äußerste Elektrode. Auch die anderen Elektroden sind direkt durch die beiden benachbarten Abstandhalter 4, 4a, 6, 6a eingespannt. Die individuelle Einspannung jeder Elektrode wird mit Bezug zu den Figs. 9 bis 12 genauer erläutert.

Zwischen den beiden Abschlussflanschen 8,10 ergibt sich somit ein kreiszylindrischer Aufbau aus den Elektroden und den Abstandhalter 4, 4a, 6, 6a, der beim gezeigten Ausführungsbeispiel von einer metallischen Überwurfhülse 12 umgriffen wird, die auch entfallen kann. Diese ist einseitig offen, so dass die elektrischen Kontakte 2 herausstehen können um mit einer übergeordneten Steuereinheit des Dialysegerätes elektrisch verbunden zu werden. Die Überwurfhülse 12 stelle eine Montagehilfe und/oder Abstützung dar. Weiterhin kann sie in Verbindung mit einem Zusatzteil (Adapterblech oder im Spritzgussverfahren hergestellter Adapter) eine Befestigungsmöglichkeit darstellen.

Bei der gezeigten Leitfähigkeitssonde hingegen sind an dem (in Figur 1 unteren) Abschlussflansch 10 sind Befestigungsösen zur Befestigung der Leitfähigkeitssonde 1 an einem Geräterahmen oder Hydraulikkomponenten des (nicht gezeigten) Dialysegerätes einstückig gebildet.

An beiden Abschlussflanschen 8, 10 sind jeweilige Schlauchtüllen 14 einstückig gebildet. Eine Schlauchtülle dient als Zulauf und die andere Schlauchtülle 14 als Ablauf (für Permeat und Bikarbonat-Konzentrat oder) für die fertige Dialysierflüssigkeit.

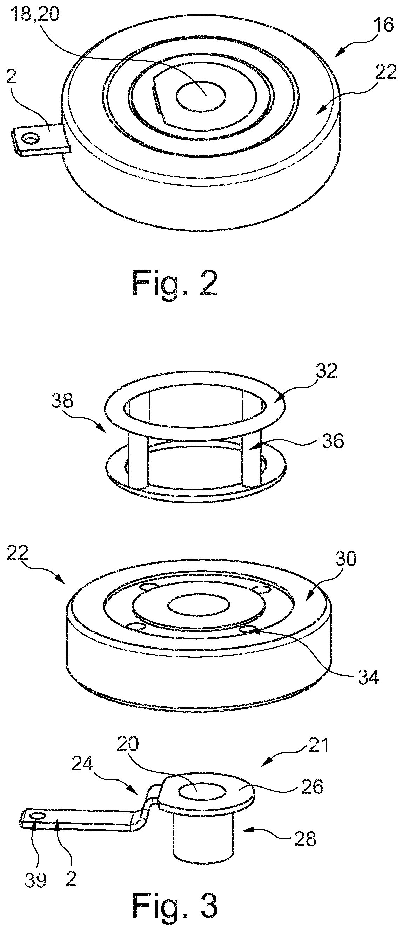

Figur 2 zeigt ein erstes Beispiel einer Elektrode 16. Sie hat eine Durchgangsausnehmung 18 mit einem elektrisch leitfähigen Innenmantel 20, der von der zu messenden Dialysierflüssigkeit oder von deren Komponenten durchströmbar ist. Die Elektrode 16 hat weiterhin einen Hauptkörper 22, an dessen Außenumfang der elektrische Kontakt 2 angeordnet ist. Der Innenmantel 20 und der elektrische Kontakt 2 sind gemeinsam aus einem ersten Material gebildet, das elektrisch leitend ist.

Figur 3 zeigt die drei wesentlichen Komponenten, aus denen die Elektrode 16 aus Fig. 2 zusammengesetzt ist. Eine Komponente ist ein Stanz-Biege-Bauteil 21, das einstückig aus dem elektrischen Kontakt 2, einem etwa s- oder stufenförmigen Verbindungsabschnitt 24, und einem Flansch 26 und einem Zylinder 28 zusammengesetzt ist. Die drei Abschnitte 2, 24, 26, 28 sind in dieser Reihenfolge aneinander befestigt, wobei der Flansch 26 an einem Endabschnitt des Zylinders 28 angeordnet ist. Damit wird eine der beiden Mündungen der Durchgangsausnehmung 18 gebildet. Der Flansch 26 ist bündig in eine der beiden (in Fig. 3 unteren) Stirnseiten 30 des Hauptkörpers 22 eingepasst. In dem Zylinder 28 ist der Innenmantel 20 gebildet, der mit dem Permeat und Bikarbonat-Konzentrat oder mit der fertige Dialysierflüssigkeit in Kontakt kommt.

Das elektrisch leitende Stanz-Biege-Bauteil 21 ist von dem Hauptkörper 22 umgeben, wobei der elektrische Kontakt 2 aus dem Hauptkörper 22 herausragt. Der Hauptkörper 22 ist bei der Elektrode 16 aus den Figs. 1 und 2 als Kunststoff-SpritzgussTeil ausgeführt, das um das Stanz-Biege-Bauteil 21 herum gespritzt wurde.

An jeder Stirnseite 30 des Hauptkörpers 22 ist eine umlaufende Nut für einen jeweiligen Dichtring 32 gebildet, wobei die beiden Nuten durch vier gleichmäßig am Umfang verteilte Verbindungslöcher 34 miteinander verbunden sind.

In einem letzten Fertigungsschritt der Elektrode 16 werden die beiden Dichtringe 32 aus Silikon oder einem anderen Dichtwerkstoff in einem Schuss an den Hauptkörper 22 angespritzt. Dabei dingt das Silikon auch durch die vier Verbindungslöcher 34 und verbindet so die beiden Dichtringe 32. Es entsteht das in Figur 3 oben gezeigte einstückige Dichtbauteil 38. Damit sind die beiden Dichtringe 32 formschlüssig an den jeweiligen Stirnseiten 30 des Hauptkörpers 22 befestigt.

Der Hauptkörper 22 und das Dichtbauteil 38 sind im Mehrkomponenten-Spritzgussverfahren hergestellt. Das zweite z.B. nicht leitende Material des Hauptkörpers 22 und das Silikon oder der andere Dichtwerkstoff des Dichtbauteils 38 sind biokompatibel.

Das Stanz-Biege-Bauteil 21 ist z.B. aus Kupfer oder Messing oder zusätzlich komplett oder partiell leitfähig biokompatibel beschichtet, z.B. mit Hartgold oder Graphit. Dies dient der Optimierung der Detektierung bzw. Signalleitung.

Der gezeigte elektrische Kontakt 2 ist ein Flachkontakt oder Steckkontakt. Der elektrische Kontakt 2 weist auch ein als Durchkontaktierung für eine Litze dienendes Durchgangsloch 39 aufweisen.

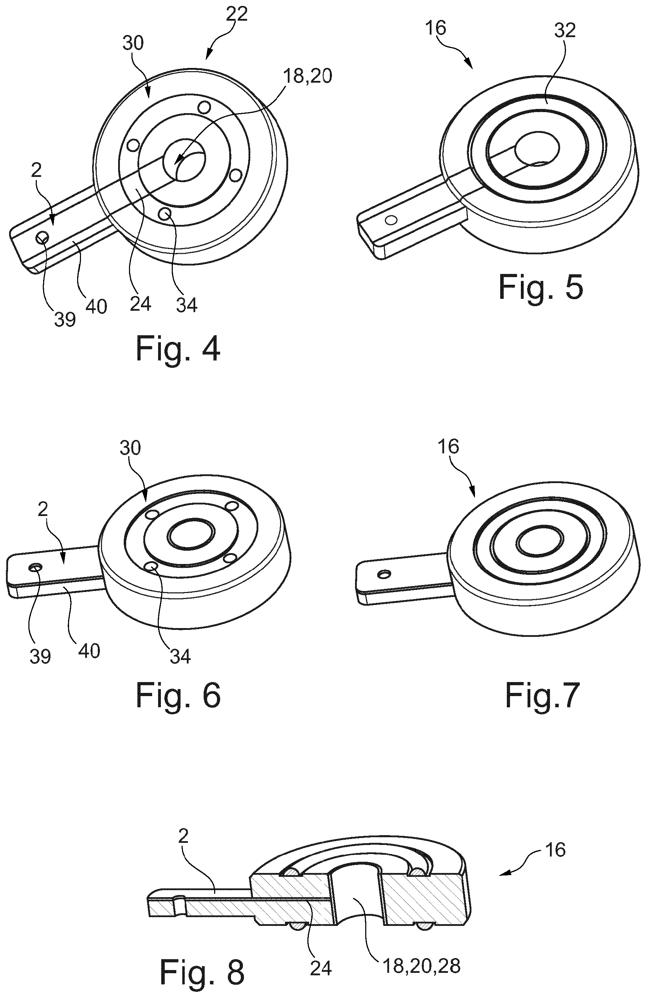

Figs. 4 und 5 zeigen ein weiteres Beispiel einer Elektrode 16, wobei Fig. 4 einen Zwischenstand der Fertigung zeigt, während die Ansicht aus Fig. 5 die fertige Elektrode 16 zeigt. Es handelt sich um eine mit einem MID-Verfahren (moulded interconnect device) oder einer ähnlichen Beschichtungstechnik hergestellte Elektrode 16. Dabei wird zunächst der Hauptkörper 22 mit einer Kontaktstütze 40 einstückig aus dem zweiten z.B. nicht leitfähigen Material (z.B. PPE+PS oder PEI oder PSU) biokompatibel gefertigt, wobei sich die Kontaktstütze 40 radial weg vom Hauptkörper 22 erstreckt.

Danach wird der elektrische Kontakt 2 mit dem Verbindungsabschnitt 24 und mit dem Innenmantel 20 aus dem leitfähigen Material, z.B. Graphit, oder Edelstahl oder Gold aufgetragen. Genauer gesagt wird der elektrische Kontakt 2 auf der Kontaktstützte 40 und der Verbindungsabschnitt 24 auf einer der Stirnseite 30 des Hauptabschnitts 22 und der Innenmantel 20 in die Durchgangsausnehmung 18 des Hauptkörpers 22 aufgetragen.

Die elektrische Kontakt 2 ist schmaler als die Kontaktstütze 40. Der Verbindungsabschnitt 24 folgt der Form der Stirnseite 30, also auch der Form der Nut für den Dichtring 32. Es ist dargestellt, dass der elektrische Kontakt 2 mit dem Verbindungsabschnitt 24 gemeinsam die Form eines durchgehenden Streifens haben.

Weiterhin abweichend von der Elektrode 16 aus Fig. 3 ist für die Ausbildung des Innenmantels 20 kein stabiler Zylinder vorgesehen. Vielmehr haben der Innenmantel 20 und der Verbindungsabschnitt 24 und der elektrische Kontakt 2 eine geringe und vorzugsweise gleiche Dicke.

Der elektrische Kontakt 2 kann als Lötpad dienen und/oder es wir das gezeigte Durchgangsloch 39 zur Befestigung einer Litze genutzt.

Figs. 6 bis 8 zeigen ein weiteres Beispiel einer Elektrode 16, wobei Fig. 6 einen Zwischenstand der Fertigung zeigt, während die Ansicht aus Fig. 7 und die geschnittene Darstellung aus Fig. 8 die fertige Elektrode 16 zeigen. In diesem Fall wurde mittels eines additiven Verfahrens (3D-Druck) die Kombination aus dem elektrisch leitfähigen Bauteil (elektrischer Kontakt 2, Verbindungsabschnitt 24 und Zylinder 28) zusammen mit dem Hauptkörper 22 aus Kunststoff zeitgleich bzw. zusammen aufgebaut (gedruckt). Danach wurde in der mit Bezug zu Figur 3 beschriebenen Weise das Dichtbauteil 38 in einem Schuss gespritzt.

Mit Blick nur auf Figs. 7 und 8 ist auch ein anderes Herstellungsverfahren für die Elektrode 16 nachvollziehbar: demnach können auch alle wesentlichen Komponenten, also der Hauptkörper 22 mit dem elektrisch leitfähigen Bauteil und mit zwei einzelnen Dichtringen 32 oder mit dem Dichtbauteil 38 mittels eines additiven Verfahrens (3D-Druck) zeitgleich bzw. in einem Vorgang aufgebaut (gedruckt) werden.

Fig. 8 zeigt, dass der elektrische Kontakt 2 und der Verbindungsabschnitt 24 in Form einer streifen- oder zungenförmigen durchgehenden Steges ausgebildet sind, der sich von einer (in Längsrichtung des Zylinders 28 betrachtet) mittleren Stelle von dem Zylinder 28 weg erstreckt.

Bei allen Elektroden 16 der Figs. 2 bis 8 können statt des in einem Schuss angespritzte Dichtbauteils 38 auch zwei einzelne Dichtringe 32 (ohne Verbindungselement 36) vorgesehen sein, die in den umlaufenden Nuten der Stirnseiten 30 angeordnet sind.

Fig. 9 zeigt eine lange und eine kurze Version eines ersten Beispiels eines Abstandhalters 4, 6 aus Fig. 1 mit der Elektrode 16 aus Figs. 2 und 3 in einer Explosionszeichnung.

Jeder Abstandhalter 4, 6 hat einen in einem Hals 42 gebildeten Durchgangskanal. Beim kürzeren Abstandhalter 4 ist der Hals 42 so kurz, dass nur eine umlaufende Nut zu erkennen ist. An jedem Endabschnitt des Halses 42 ist ein als Flansch 44 ausgestalteter Anlagebereich vorgesehen. An den Flanschen 44 ist eine Anlagefläche 46 für eine Stirnseite 30 einer Elektrode 16 gebildet.

Die Anlageflächen 46 haben jeweils einen weitgehend umlaufenden Außenrand 48, der durch eine Ausnehmung (Kerbe) für den elektrischen Kontakt 2 der betroffene Elektrode 16 unterbrochen ist. An den beiden Außenrändern 48 sind jeweils ein als Ringschnappverbindung dienender äußerer Wulst 50 oder eine als Ringschnappverbindung dienende innere Nut 52 gebildet.

Gemäß dieser Schrift ist also eine Ringschnappverbindung oder auch eine (nicht gezeigte) Clipsverbindung als einseitige Vorrichtung zu verstehen. Zwei derartige Vorrichtungen bilden zusammen eine Einspannung für die betroffene Elektrode 16.

In jedem Flansch 44 sind zwei außermittige Querbohrungen vorgesehen, die als Bügellöcher 54 dienen, und die mit Bezug zu Fig. 10 genauer erläutert werden.

Fig. 10 zeigt die Anordnung aus Fig. 9 im zusammengesetzten Zustand. Zusätzlich sind außen noch zwei weitere Elektroden 16 gezeigt. Es sind die drei Beispiele der Elektroden 16 aus den Figs. 2 bis 8 kombiniert, um die modulare Austauschbarkeit bzw. Kompatibilität der Elektroden 16 mit den Abstandhaltern 4, 6 zu zeigen.

Die mittlere Elektrode, von der nur der elektrische Kontakt 2 zu sehen ist, hat eine Einspannung. Die Einspannung ist von der inneren Nut 52 (erste Ringschnappverbindung) und dem darin eingeschnappten äußeren Wulst 50 (zweite Ringschnappverbindung) gebildet. Durch den weitgehend und fast geschlossenen Umlauf des Wulstes 50 und der Nut 52 am Außenrand 48 ist eine stabile Befestigung der Abstandhalter 4, 6 aneinander und eine dichte Einspannung der Elektrode erreicht.

Eine optionale Sicherung ist durch zwei metallische Bügel 56 realisiert, von denen in Figs. 9 und 10 jeweils nur ein Bügel 56 gezeigt ist. Die Bügel 56 haben jeweils zwei etwa parallel Schenkel 58, die in die Bügellöcher 54 zweier aneinander anliegende Flansche 44 eingesteckt werden. Wenn die beiden Schenkel 58 eines Bügels 56 gegeneinander gespannt sind, ist eine zweite Einspannung gegeben.

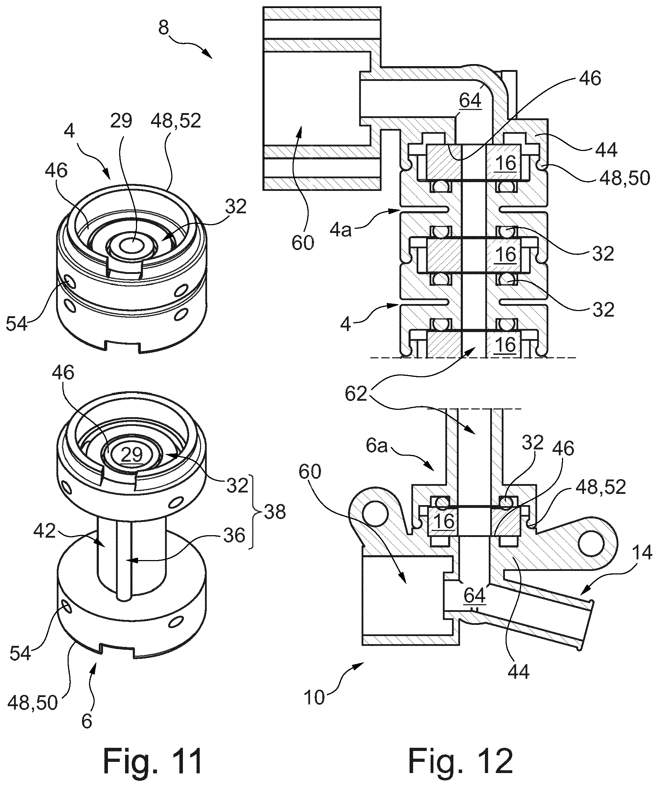

Fig. 11 zeigt eine lange und eine kurze Version eines zweiten Beispiels eines Abstandhalters 4, 6. Dabei entsprechen die Außenränder 48 mit den als Wulst 50 und als Nut 52 gebildeten Ringschnappverbindungen und den zusätzlichen Bügellöchern 54 denjenigen der Abstandhalter 4, 6 aus Figs. 9 und 10.

Wenn die Abstandhalter 4, 6 gemäß Fig. 11 mit den Elektroden 16 der Figs. 2 bis 8 kombiniert werden, dann entfallen deren Dichtringe 32 bzw. Dichtbauteile 38, denn es sind Dichtringe 32 an den Abstandhaltern 4, 6 vorgesehen. Es können also die mit Bezug zu den Figs. 2 bis 8 beschriebenen Aufbauten der Elektroden 16 aus elektrisch leitendem Bauteil und Hauptkörper 22 mit den dort genannten Herstellungsverfahren allerdings ohne Dichtringe 32 bzw. Dichtbauteile 38 in die Abstandhalter 4, 6 gemäß Fig. 11 eingespannt werden.

Bei den Abstandhaltern 4, 6 gemäß Fig. 11 ist an jeder Anlagefläche 46 ein Dichtring 32 vorgesehen. Die beiden Dichtringe 32 sind über zwei Verbindungselemente 36 miteinander einstückig verbunden, von denen in Fig. 11 nur ein Verbindungelement 36 des längeren Abstandhalters 6 zu erkennen ist. Die Verbindungselemente 36 erstrecken sich an der Außenseite des Halses 46. Damit ist ein formschlüssig befestigtes Dichtbauteil 38 am Abstandhalter 4, 6 gebildet.

Alle gezeigten Abstandhalter 4, 6 können im Kunststoff-Spritzguss-Verfahren aus biokompatiblem PPE+PS oder PEI oder PSU hergestellt werden. Bei den Abstandhaltern 4, 6 aus Fig. 11 ist das einstückige Dichtbauteil 38 aus Silikon oder einem anderen Dichtwerkstoff in einem Schuss angespritzt.

Fig. 12 zeigt einen oberen und einen unteren Ausschnitt der Leitfähigkeitssonde 1 aus Fig. 1 mit den Abstandhaltern 4, 6 aus Fig. 11, also mit jeweiligem Dichtbauteil 38 mit zwei formschlüssig befestigten Dichtringen 32. Es sind mehrere Elektroden 16 entlang dem Messkanal aufgereiht, die keine eigenen Dichtelemente haben. Nur zwischen den beiden äußersten Elektroden 16 und dem jeweiligen Abschlussflansch 8, 10 muss noch ein extra Dichtring (nicht gezeigt) ergänzt werden.

Es ist zu erkennen, dass auch die beiden Abschlussflansche 8, 10 jeweils eine Ringschnappverbindung haben. Genauer gesagt weist der eine (in Figur 12 obere) Abschlussflansch 8 an dem Außenrand 48 seiner Anlagefläche 46 einen äußeren Wulst 50 auf, während der andere (in Figur 12 untere) Abschlussflansch 10 an dem Außenrand 48 seiner Anlagefläche 46 eine innere Nut 52 ausweist. Damit ist für alle Elektroden 16 also auch für die beiden äußersten Elektroden 16 jeweils eine individuelle Einspannung, die bei der Leitfähigkeitssonde 1 gemäß Fig. 12 alle von Ringschnappverbindungen, genauer gesagt von äußeren Wülsten 50 oder innere Nuten 52 gebildet sind.

Die beiden Abschlussflansche 8 ,10 verbinden jeweils einen Endabschnitt des aus den Durchgangsausnehmungen 18 der Elektroden 16 und den Durchgangskanälen der Abstandhalter 4, 6 gebildeten Messkanal 62 über eine abgewinkelten Kanal 64 mit dem Dialysegerät. An jedem abgewinkelten Kanal 64 ist ein Sensoraufnahme 60 gebildet. Dort kann ein Sensor z.B. für Temperatur, Druck und/oder Flussgeschwindigkeit eingesetzt werden.

### Bezugszeichenliste

- 1: Leitfähigkeitssonde
- 2: elektrischer Kontakt
- 4: kürzerer Abstandhalter
- 4a: äußerster kürzerer Abstandhalter
- 6: längerer Abstandhalter
- 6a: äußerster längerer Abstandhalter
- 8: Abschlussflansch
- 10: Abschlussflansch
- 12: Überwurfhülse
- 14: Schlauchtülle
- 16: Elektrode
- 18: Durchgangsausnehmung
- 20: Innenmantel
- 21: Stanz-Biege-Bauteil
- 22: Hauptkörper
- 24: Verbindungsabschnitt
- 26: Flansch
- 28: Zylinder
- 29: Mündung des Durchgangskanals
- 30: Stirnseite
- 32: Dichtring
- 34: Verbindungsloch
- 36: Verbindungselement
- 38: Dichtbauteil
- 39: Durchgangsloch
- 40: Kontaktstütze
- 42: Hals
- 44: Analgebereich / Flansch
- 46: Anlagefläche
- 48: Außenrand
- 50: äußerer Wulst (Spannvorrichtung)
- 52: innere Nut (Spannvorrichtung)
- 54: Bügelloch (Spannvorrichtung)
- 56: Bügel (Spannvorrichtung)
- 58: Schenkel (Spannvorrichtung)
- 60: Sensoraufnahme
- 62: Messkanal
- 64: abgewinkelter Kanal

## Patentansprüche

1. Leitfähigkeitssonde (1) mit mindestens einem Abstandhalter (4, 4a, 6, 6a) und mit mindestens zwei Elektroden (16), wobei die Anzahl der Elektroden (16) um eins größer ist, als die Anzahl der Abstandhalter (4, 4a, 6, 6a), wobei an den beiden äußersten Elektroden (16) innenseitig der genau eine Abstandhalter oder ein äußerster Abstandhalter (4a, 6a) und außenseitig ein Abschlussflansch (8, 10) angeordnet sind, wobei der Abstandhalter (4, 4a, 6, 6a) einen Durchgangskanal mit zwei Mündungen (29) aufweist, wobei an jeder Mündung (29) ein Anlagebereich (44) vorgesehen ist, der eine die Mündung (29) umgebende Anlagefläche (46) aufweist, wobei auch die beiden Abschlussflansche (8, 10) jeweils einen Anlagebereich (44) mit einer Anlagefläche (46) aufweisen, wobei die beiden äußersten Elektroden (16) jeweils zwischen den beiden aufeinander zuweisenden Anlageflächen (46) des genau einen oder des äußersten Abstandhalters (4a, 6a) und des Abschlussflansches (8, 10) dichtend eingespannt sind, wobei an den beiden Anlagebereichen (44) des Abstandhalters (4, 4a, 6, 6a) jeweils eine Spannvorrichtung (50, 52; 54, 56, 58) vorgesehen ist, wobei die beiden Spannvorrichtungen (50, 52; 54, 56, 58) unabhängig voneinander in Wirkverbindung mit einer jeweiligen Spannvorrichtung (50, 52; 54, 56, 58) eines Anlagebereiches (44) eines weiteren Abstandhalters (4, 4a, 6, 6a) oder Abschlussflansches (8, 10) der Leitfähigkeitssonde (1) bringbar sind, wobei an den Anlagebereichen (44) der beiden Abschlussflansche (8, 10) jeweils eine Spannvorrichtung (50, 52; 54, 56, 58) vorgesehen ist, wobei die Spannvorrichtungen (50, 52; 54, 56, 58) unabhängig voneinander aktivierbar und deaktivierbar sind und unabhängig voneinander in Wirkverbindung mit der benachbarten Spannvorrichtung (50, 52; 54, 56, 58) des Anlagebereiches (44) des genau einen oder des jeweiligen äußersten Abstandhalters (4a, 6a) bringbar sind.

2. Leitfähigkeitssonde (1) nach Anspruch 1, wobei die beiden Anlageflächen (46) des Abstandhalters (4, 4a, 6, 6a) jeweilige Außenränder (48) haben, **dadurch gekennzeichnet, dass** die beiden Außenränder (48) rund oder eckig sind.

3. Leitfähigkeitssonde (1) nach einem der vorhergehenden Ansprüche, **dadurch gekennzeichnet, dass** an zumindest einer der Anlageflächen (46) des Abstandhalters (4, 4a, 6, 6a) ein Dichtring (32) angeordnet ist, der die an der Anlagefläche (46) gebildete Mündung (29) des Durchgangskanals vollumfänglich umgibt, wobei der Dichtring (32) stoffschlüssig an der Anlagefläche (46) befestigt ist.

4. Leitfähigkeitssonde (1) nach Anspruch 1 oder 2, **dadurch gekennzeichnet, dass** an beiden Anlageflächen (46) des Abstandhalters (4, 4a, 6, 6a) jeweils ein Dichtring (32) angeordnet ist, der die jeweilige Mündung (29) des Durchgangskanals vollumfänglich umgibt, wobei die beiden Dichtringe (32) gemeinsam mit mindestens einem Verbindungselement (36) ein einstückiges Dichtbauteil (38) bilden, wobei sich das Verbindungselement (36) zwischen den beiden Dichtringen (32) erstreckt.

5. Leitfähigkeitssonde (1) nach einem der vorhergehenden Ansprüche, **dadurch gekennzeichnet, dass** die beiden Anlagebereiche (44) des Abstandhalters (4, 4a, 6, 6a) jeweils von einem Flansch gebildet sind, wobei sich zwischen den beiden Flanschen ein Hals (42) mit gegenüber den beiden Flanschen vermindertem Durchmesser erstreckt, wobei in dem Hals (42) ein mittlerer Abschnitt des Durchgangskanals gebildet ist.

6. Leitfähigkeitssonde (1) nach einem der vorhergehenden Ansprüche, wobei die Spannvorrichtung (50, 52) jeweils eine Seite einer Ringschnappverbindung ist oder umfasst.

7. Leitfähigkeitssonde (1) nach Anspruch 6, wobei die Ringschnappverbindung kreis- oder kreisbogenförmig ausgestaltet ist und sich vollumfänglich oder weitgehend oder abschnittsweise an einem jeweiligen Außenrand (48) erstreckt.

8. Leitfähigkeitssonde (1) nach Anspruch 7, **dadurch gekennzeichnet, dass** die Ringschnappverbindung einen zumindest abschnittsweise umlaufenden äußeren Wulst (50) an dem einen Außenrand (48) und eine zumindest abschnittsweise umlaufende innere Nut (52) an dem anderen Außenrand (48) ist oder aufweist.

9. Leitfähigkeitssonde (1) nach einem der Ansprüche 1 bis 5, **dadurch gekennzeichnet, dass** die Spannvorrichtung jeweils eine Seite mindestens einer Clipsverbindung ist oder umfasst.

10. Leitfähigkeitssonde (1) nach einem der Ansprüche 1 bis 5, **dadurch gekennzeichnet, dass** die Spannvorrichtung (54, 56, 58) jeweils beidseitig der Mündung (29) des Durchgangskanals je ein Bügelloch (54) umfasst, wobei in eines der Bügellöcher (54) ein Schenkel (58) eines u-förmigen Bügels (56) eingesetzt ist, während in das andere Bügelloch (54) ein Schenkel (58) eines weiteren u-förmigen Bügels (56) des weiteren Abstandhalters (4, 6) einsetzbar ist.

11. Leitfähigkeitssonde (1) nach einem der vorhergehenden Ansprüche, **dadurch gekennzeichnet, dass** am Außenumfang des mindestens einen Abstandhalters (4, 4a, 6, 6a) und der mindestens zwei Elektroden (16) eine Überwurfhülse (12) angeordnet ist.

12. Leitfähigkeitssonde (1) nach einem der vorhergehenden Ansprüche, **dadurch gekennzeichnet, dass** an zumindest einem der beiden Abschlussflansche (8, 10) eine Schlauchtülle (14) und/oder eine Sensoraufnahme (60) und/oder ein Befestigungsmittel zur Befestigung der Leitfähigkeitssonde (1) an dem Dialysegerät gebildet sind/ist.

## Claims

1. A conductivity probe (1) having at least one spacer (4, 4a, 6, 6a) and having at least two electrodes (16), wherein the number of electrodes (16) is greater by one than the number of spacers (4, 4a, 6, 6a), wherein the precisely one spacer or an outermost spacer (4a, 6a) is arranged on the inner side of the two outermost electrodes (16) and a terminating flange (8, 10) is arranged on the outer side, wherein the spacer (4, 4a, 6, 6a) has a through-channel with two orifices (29), wherein a contact region (44) is provided at each orifice (29), wherein the contact region (44) has a contact surface (46) surrounding the orifice (29), wherein the two terminating flanges (8, 10) also each have a contact region (44) with a contact surface (46), wherein the two outermost electrodes (16) are each clamped in a sealing manner between the two contact surfaces (46) of the precisely one spacer or of the outermost spacer (4a, 6a) and of the terminating flange (8, 10) that face towards each other, wherein a respective clamping device (50, 52; 54, 56, 58) is provided in each case at the two contact regions (44) of the spacer (4, 4a, 6, 6a), wherein the two clamping devices (50, 52; 54, 56, 58) can be brought into operative connection independently of each other with a respective clamping device (50, 52; 54, 56, 58) of a contact region (44) of a further spacer (4, 4a, 6, 6a) or of a terminating flange (8, 10) of the conductivity probe (1), wherein a respective clamping device (50, 52; 54, 56, 58) is provided in each case at the contact regions (44) of the two terminating flanges (8, 10), wherein the clamping devices (50, 52; 54, 56, 58) can be activated and deactivated independently of each other and can be brought into operative connection independently of each other with the adjacent clamping device (50, 52; 54, 56, 58) of the contact region (44) of the precisely one spacer or of the respective outermost spacer (4a, 6a).

2. The conductivity probe (1) according to claim 1, wherein the two contact surfaces (46) of the spacer (4, 4a, 6, 6a) have respective outer edges (48), **characterized in that** the two outer edges (48) are round or angular.

3. The conductivity probe (1) according to one of the preceding claims, **characterized in that** a sealing ring (32) is arranged on at least one of the contact surfaces (46) of the spacer (4, 4a, 6, 6a), wherein the sealing ring completely surrounds the orifice (29) of the through-channel formed on the contact surface (46), wherein the sealing ring (32) is fastened to the contact surface (46) in a materially bonded manner.

4. The conductivity probe (1) according to Claim 1 or 2, **characterized in that** a respective sealing ring (32) is arranged in each case at the two contact surfaces (46) of the spacer (4, 4a, 6, 6a), wherein the sealing ring fully surrounds the respective orifice (29) of the through-channel, wherein the two sealing rings (32) together with at least one connecting element (36) form a one-piece sealing component (38), wherein the connecting element (36) extends between the two sealing rings (32).

5. The conductivity probe (1) according to one of the preceding claims, **characterized in that** the two contact regions (44) of the spacer (4, 4a, 6, 6a) are formed in each case by a flange, wherein a neck (42) with a reduced diameter in relation to the two flanges extends between the two flanges, wherein a central section of the through-channel is formed in the neck (42).

6. The conductivity probe (1) according to one of the preceding claims, wherein the clamping device (50, 52) is or comprises in each case one side of an annular snap connection.

7. The conductivity probe (1) according to Claim 6, wherein the annular snap connection is of circular or arcuate configuration and extends fully circumferentially or largely or in sections at a respective outer edge (48).

8. The conductivity probe (1) according to Claim 7, **characterized in that** the annular snap connection is or has an at least partially circumferential outer bead (50) at the one outer edge (48) and an at least partially circumferential inner groove (52) at the other outer edge (48).

9. The conductivity probe (1) according to one of Claims 1 to 5, **characterized in that** the clamping device is or comprises in each case one side of at least one clip connection.

10. The conductivity probe (1) according to one of Claims 1 to 5, **characterized in that** the clamping device (54, 56, 58) comprises one respective bracket hole (54) in each case on both sides of the orifice (29) of the through-channel, wherein a leg (58) of a U-shaped bracket (56) is inserted into one of the bracket holes (54), while a leg (58) of a further U-shaped bracket (56) of the further spacer (4, 6) is insertable into the other bracket hole (54).

11. The conductivity probe (1) according to one of the preceding claims, **characterized in that** a union sleeve (12) is arranged on the outer circumference of the at least one spacer (4, 4a, 6, 6a) and of the at least two electrodes (16).

12. The conductivity probe (1) according to one of the preceding claims,
**characterized in that** a hose nozzle (14) and/or a sensor receptacle (60) and/or a fastening means for fastening the conductivity probe (1) to the dialysis machine are/is formed on at least one of the two end flanges (8, 10).

## Revendications

1. Sonde de conductivité (1) avec au moins un espaceur (4, 4a, 6, 6a) et avec au moins deux électrodes (16), dans laquelle le nombre d'électrodes (16) est supérieur d'un au nombre des espaceurs (4, 4a, 6, 6a), dans laquelle le précisément un espaceur ou un espaceur le plus extérieur (4a, 6a) sont agencés côté intérieur au niveau des deux électrodes les plus extérieures (16), et une bride d'extrémité (8, 10) est agencée côté extérieur, dans laquelle l'espaceur (4, 4a, 6, 6a) présente un canal débouchant avec deux embouchures (29), dans laquelle une zone d'appui (44) qui présente une surface d'appui (46) entourant l'embouchure (29) est prévue au niveau de chaque embouchure (29), dans laquelle les deux brides d'extrémité (8, 10) présentent aussi respectivement une zone d'appui (44) avec une surface d'appui (46), dans laquelle les deux électrodes les plus extérieures (16) sont serrées de manière étanche respectivement entre les deux surfaces d'appui (46) tournées l'une vers l'autre du précisément un ou de l'espaceur (4a, 6a) le plus extérieur et de la bride d'extrémité (8, 10), dans laquelle respectivement un dispositif de serrage (50, 52 ; 54, 56, 58) est prévu au niveau des deux zones d'appui (44) de l'espaceur (4, 4a, 6, 6a), dans laquelle les deux dispositifs de serrage (50, 52 ; 54, 56, 58) peuvent être amenés indépendamment l'un de l'autre en liaison active avec un dispositif de serrage respectif (50, 52 ; 54, 56, 58) d'une zone d'appui (44) d'un autre espaceur (4, 4a, 6, 6a) ou d'une autre bride d'extrémité (8, 10) de la sonde de conductivité (1), dans laquelle respectivement un dispositif de serrage (50, 52 ; 54, 56, 58) est prévu au niveau des zones d'appui (44) des deux brides d'extrémité (8, 10), dans laquelle les dispositifs de serrage (50, 52 ; 54, 56, 58) peuvent être activés et désactivés indépendamment les uns des autres et peuvent être amenés indépendamment les uns des autres en liaison active avec le dispositif de serrage contigu (50, 52 ; 54, 56, 58) de la zone d'appui (44) du précisément un ou de l'espaceur (4a, 6a) le plus extérieur respectif.

2. Sonde de conductivité (1) selon la revendication 1, dans laquelle les deux surfaces d'appui (46) de l'espaceur (4, 4a, 6, 6a) présentent des bords extérieurs (48) respectifs, **caractérisée en ce que** les deux bords extérieurs (48) sont ronds ou anguleux.

3. Sonde de conductivité (1) selon l'une quelconque des revendications précédentes, **caractérisée en ce qu'**une bague d'étanchéité (32) est agencée au niveau d'au moins l'une des surfaces d'appui (46) de l'espaceur (4, 4a, 6, 6a), bague qui entoure intégralement l'embouchure (29) du canal débouchant formée au niveau de la surface d'appui (46), dans laquelle la bague d'étanchéité (32) est fixée par complémentarité de matières à la surface d'appui (46).

4. Sonde de conductivité (1) selon la revendication 1 ou 2, **caractérisée en ce que** respectivement une bague d'étanchéité (32) est agencée au niveau des deux surfaces d'appui (46) de l'espaceur (4, 4a, 6, 6a), bague qui entoure intégralement l'embouchure (29) respective du canal débouchant, dans laquelle les deux bagues d'étanchéité (32) forment conjointement avec au moins un élément de liaison (36) un composant d'étanchéité (38) d'un seul tenant, dans laquelle l'élément de liaison (36) s'étend entre les deux bagues d'étanchéité (32).

5. Sonde de conductivité (1) selon l'une quelconque des revendications précédentes, **caractérisée en ce que** les deux zones d'appui (44) de l'espaceur (4, 4a, 6, 6a) sont formées respectivement par une bride, dans laquelle un col (42) avec un diamètre réduit par rapport aux deux brides s'étend entre les deux brides, dans laquelle une section centrale du canal débouchant est formée dans le col (42).

6. Sonde de conductivité (1) selon l'une quelconque des revendications précédentes, dans laquelle le dispositif de serrage (50, 52) est ou comprend respectivement un côté d'une liaison d'enclenchement par bague.

7. Sonde de conductivité (1) selon la revendication 6, dans laquelle la liaison d'enclenchement par bague est configurée en forme de cercle ou d'arc de cercle et s'étend intégralement ou largement ou par sections au niveau d'un bord extérieur (48) respectif.

8. Sonde de conductivité (1) selon la revendication 7, **caractérisée en ce que** la liaison d'enclenchement par bague est ou présente un bourrelet (50) extérieur périphérique au moins par sections au niveau de l'un bord extérieur (48) et une rainure (52) intérieure périphérique au moins par sections au niveau de l'autre bord extérieur (48).

9. Sonde de conductivité (1) selon l'une quelconque des revendications 1 à 5, **caractérisée en ce que** le dispositif de serrage est ou comprend respectivement un côté au moins d'une liaison par clip.

10. Sonde de conductivité (1) selon l'une quelconque des revendications 1 à 5, **caractérisée en ce que** le dispositif de serrage (54, 56, 58) comprend respectivement des deux côtés de l'embouchure (29) du canal débouchant chaque fois un trou d'étrier (54), dans laquelle une branche (58) d'un étrier (56) en forme de u est insérée dans l'un des trous d'étrier (54), alors qu'une branche (58) d'un autre étrier (56) en forme de u de l'autre espaceur (4, 6) peut être insérée dans l'autre trou d'étrier (54).

11. Sonde de conductivité (1) selon l'une quelconque des revendications précédentes, **caractérisée en ce qu'**un manchon (12) est agencé au niveau de la périphérie extérieure de l'au moins un espaceur (4, 4a, 6, 6a) et des au moins deux électrodes (16).

12. Sonde de conductivité (1) selon l'une quelconque des revendications précédentes, **caractérisée en ce qu'**un embout de tuyau (14) et/ou un logement de capteur (60) et/ou un moyen de fixation pour la fixation de la sonde de conductivité (1) est/sont formés sur l'appareil de dialyse au niveau d'au moins l'une des deux brides d'extrémité (8, 10).
